# EUROPEAN PATENT APPLICATION

(11) **EP 2 647 656 A2**
(43) Date of publication of application: **09.10.2013**
(21) Application number: 12189833.2
(22) Date of filing: 24.10.2012
(51) Int. Cl.: C08G 63/08, C08G 63/64, C08G 63/664

(54) **Bioresorbable and biocompatible thermoplastic elastomer having a shape memory, particularly for biomedical applications and a process for their preparation**

(30) Priority: 02.04.2012 PL 39868712
(71) Applicant: Centrum Materialów Polimerowych i Weglowych PAN, 41-800 Zabrze (PL)
(72) Inventor: Dobrzy ski, Piotr, 41-808 Zabrze (PL); Kasperczyk, Janusz, 40-693 Katowice (PL); Smola, Anna, 41-908 Bytom (PL); Pastusiak, Ma gorzata, 41-803 Zabrze (PL); Sobota, Micha, 42-224 Cz stochowa (PL); Jaworska, Joanna, 41-710 Ruda l. (PL)
(74) Representative: Brodowska, Iwona

(57) **Abstract**

Bioresorbable, fully biocompatible thermoplastics, especially amorphous, which are aliphatic copolyesters or aliphatic poly(ester-co-carbonate) or poly(ester-co-amide), and poly(ester-co-carbonate-co-amide) or poly(amide-co-carbonate), which exhibit elastic properties at temperatures a little higher than the temperature of the human body in the range of 38 - 45°C, and at lower temperatures (in the range of 0° to 38°C) with a relatively high modulus of rigidity, of the controlled synthesis conditions of morphology and the shape memory properties, including long homoblocks and short mixed sequences derived from comonomers used, having the formula 7,

-(AAAA)ₙ-(ABAB)ₖ-(BBBB)ₘ- Formula 7

where n, k and m represent a natural number from 2 to 200, the component of unit A is a group of formula -O-CH(R)-CO-, -O-(CHR)_{4···6}-CO- and / or -O-(CHR)_{4···6} -O-CO-, in which R is -(CH₂)ₐR₁ or -(CHR₂-OCH₂)_{b}-OH or -(CHR₂-OCH₂)_{b}-NH₂, where a is an integer from 0 to 10, b is an integer from 1 to 1000, R₁ is -H or -OH or -NH₂, R₂ is -CH₃ or -CH₂CH₃, and the component of unit B is a group of formula -O-CH₂-CO-, and/or -O-(CH₂)_{4···6}-CO-, and/or -O(CH₂)_{4···6}-O-CO-. The present invention relates to a process for preparation of bioresorbable polymeric materials with shape memory of the process consists in carrying out the copolymerization, running according to the known mechanism of ROP, the above-mentioned monomers. t-butoxy zirconium (IV), titanium acetylacetonate (IV).

## Description

### Technical Field

This invention relates to bioresorbable, fully biocompatible thermoplastic, especially amorphous, which are aliphatic copolyesters or aliphatic poly(ester-co-carbonate) or poly(ester-co-amide), and poly(ester-co-carbonate-co-amide), or poly(amide-co-carbonate), which exhibit elastic properties at temperatures a little higher than the temperature of the human body in the range of 38° - 45°C, and at lower temperatures (in the range of 0° to 38°C) with a relatively high modulus of rigidity, of the controlled synthesis conditions of morphology and the shape memory properties, including long homoblocks and short mixed sequences derived from comonomers used, having the formula 7,

- (AAAA)ₙ-(ABAB)ₖ-(BBBB)ₘ- Formula 7

where n, k and m represent a natural number from 2 to 200, the component of unit A is a group of formula -O-CH(R)-CO-, -O-(CHR)_{4···6} -CO- and / or -O-(CHR)_{4···6}-O-CO-, in which R is -(CH₂)ₐR₁ or -(CHR₂ -OCH₂)_{b}-OH or -(CHR₂-OCH₂)_{b}-NH₂, where a is an integer from 0 to 10, b is an integer from 1 to 1000, R₁ is -H or -OH or -NH₂, R₂ is -CH₃ or -CH₂ CH₃, and the component of unit B is a group of formula -O-CH₂-CO-, and/or -O-(CH₂)_{4···6}-CO-, and/or -O(CH₂)_{4 ···6}-O-CO-, and a process for their preparation.

### Background Art

Biodegradable polymers, especially aliphatic copolymers of lactide and / or glycolide and / or lactones such as ε-caprolactone, δ-valerolactone and /or aliphatic cyclic carbonates such as trimethylene carbonate, dimethyl trimethylene carbonate, are known and valuable biomaterials used for years in modern surgery in the form of temporary degradable implants (N. Ashammakhi, P.Rokkanen, Absorbable polyglycolide devices in trauma and bone surgery, Biomaterials, 18, 1997, 3-9. D. Eglin, M. Alina, Degradable polymeric materials for osteosynthesis: Tutorial, European Cells and Materials, 16, 2008, 80-91., SL Bennett, CH. K. Liu, U.S. Patent 5 431 679, LS Nair, CT Laurencin, Biodegradable polymers as Biomaterials, Prog. Polym. Sci. 32 ( 2007) 762-798.). The materials described above are also being used increasingly as a drug carrier, introduced and released directly into the patient's body, usually in the form of micro- and nano- capsules, containing dissolved or dispersed drug, as well as the material from which they are formed, a porous three-dimensional scaffold used for settlement and cultivation in vitro cell or tissue in tissue engineering techniques [S. Mukherjee, C. Gualandi, ML Focarete, R. Ravichandran, JR Venugopal, M. Raghunath, S. Ramakrishna, Elastomeric Scaffolds of electrospun poly (L-lactide-co-trimethylene carbonate) for myocardial tissue engineering, 22, 2001, 1689 - 1699].

Presented application generally refers to the material described above, which is achieved by the subject invention, in addition to preservation of biocompatibility and biodegradability properties - very valuable from the point of their possible biomedical applications, having high elasticity at temperatures a little higher than the temperature of the human body and a relatively high stiffness at lower temperatures. Selected in accordance with the invention, the structure and composition of the polymer chains allows that the materials obtained according to the invention may be subject to large easily controlled deformations (reaching over 300% change in the output dimension) of the original shape to the designed temporary shape, and after activation of the temperature stimulus, which can be the temperature of human body, are capable of rapid return of the given shape to the shape of the original output. This type of ownership of the materials are known and called "shape memory". The first materials known to possess the property are metal melts with shape memory (SMAs), including titanium and nickel melts, copper / zinc / aluminum [W. J. Buehler, JV Gilfrich, RC Wiley, Effect of Low-Temperature Phase Changes on the Mechanical Properties of Melts near Composition TiNi, J. Appl Phys 1963, 34, 1475-1477]. Also known are many polymers (SMPs) with this property. SMPs polymers, especially thermoplastics, in comparison with melts frying SMAs are easier to form, they may be subject to a greater deformation and the increase of temperature induced return to their initial shape is much faster and more "detailed". This observed phenomenon of changes of the shape of the polymer is because of the combination between specific molecular structure of the polymer chains SMPS, causing multi-phase morphology of the material and processing technology of the material during the broadcast preset shape and form permanent relief. To present the shape memory effect polymers must be in inter-node structure - linking polymer chains of different molecules and the structure of flexible switches, which are segments of the chain [Lendlein, A. and Kelch, S., Angew.Chem. Int Ed., 41 (12), 2034, 2002]. In the case of thermoplastic SMPs nodes may not be the chemical bonds of a covalent, it can be create only by strong intermolecular interactions of a physical (eg microfibre separation and the formation of crystalline domains, tangled chains), strong enough to avoid break in the process of shaping but low enough to maintain the plasticity of the polymer at high temperatures, and thus enable their processing by injection molding, pressing, blow molding or other known methods.

In literature thermoplastic SMPs are mainly characterized as linear block polymers whose chains contain well-defined flexible and stiff segments, which is reflected in the multi-phase morphology of the material [M. Behl, A. Lendlain, Shape-memory polymers, Materials Today, 10, 2007, 20-28]. The rigid segments of the chain form typical semi-crystalline phase, with a clearly defined melting point, and the flexible segments form part of the amorphous domains of defined low glass transition temperatures. When such a polymer is heated above the melting point of the hard segments of the crystalline phase, the material can be molded to the desired shape of the original, by conventional thermoplastic processing. After cooling and reheating above the glass transition temperature of the amorphous domain, and below the melting point of the crystalline phase, it is possible to a certain extent, the deformation of the sample formed without its destruction. Obtained temporary shape can be preserved by subsequent rapid cooling of the sample below the glass transition temperature of the amorphous domain. As a result of re-heating the sample to a temperature approaching the glass transition temperature of the amorphous domain is the phenomenon of spontaneous return of the sample to the temporary shape of the original shape.

SMPs polymers used as bioresorbable implanting material (surgical implants, drug carriers, porous scaffolds for cell culture) for reasons of utility must demonstrate the following characteristics:

- Fully biocompatible, which means that the conditions of biocompatibility must fulfill not only the finished polymer material, but also all the products of its degradation, which occurs after implantation, and any additives contained in the material to improve the processing or enabling polymerizations (colorants, stabilizers, polymerization initiators and other) release during degradation of the implanted implant,

- Should be thermoplastics, - for easy processing and the ability to obtain complex shapes,

- Should be highly flexible in the temperature of deformation during the provisional shaping - which facilitates this process, allows to obtain large changes in the dimensions of such devices, and prevents the formation of mechanical defects in the product during the process of shaping a temporary shape

- The temperature of inducing the phenomenon of reverse shape of the SMPs from the temporary shape to the permanent programmed shape should be close to the temperature of the human body and cannot be greater than 45-50°C due to the destructive effect of elevated temperature on the living tissue.

Most thermoplastic polymers synthesized SMPs currently does not meet all the conditions submitted above and more cannot be applied, as a material for molding medical implants [Hong-Yan Jiang and Annette M. Schmidt, The Structural Variety of Shape-Memory Polymers for Shape-Memory Polymers and Multifunctional Composites, ed. J. Leng, Sh. Du, CRC Press Taylor & Francis Group, Boca Raton, London, New York, 2010]. Biodegradable thermoplastic with shape memory that can be used biomedical are primarily polyurethanes [Metcalfe, A., Desfaits, AC, Salazkin, I., Yahia, L., Sokolowski, WM, Raymond, J. (2003). Cold hibernated elastic memory foams for endovascular Interventions, Biomaterials, 24, 491-497. Hayashi, S. (1993). Properties and applications of polyurethane shape memory polymer series, Int Prog. URETHANES, 6, 90-115.]. These materials, however, have a very long biodegradation time into products which are often not sufficiently biocompatible, which strongly limits their use in medicine.

For this reason, attempts to obtain biodegradable thermoplastics based on aliphatic polyesters, and polycarbonates. In these polymers are obtained multiblock chain structure comprising separate segments of rigid and long flexible segments, capable of producing a shape memory effect. Currently, biodegradable copolymers remembering shape, based on the said monomers, in order to obtain the structure of a substantially microblocks are obtained in two stages - by synthesis macrodiols derived from cyclic diesters and lactones, hydroxyl-terminated (oligolactide, oligo(ε-caprolactone), oligo-(lactide-co-glycolide)) which are then subjected to a condensation reaction with difunctional coupling compounds, such as: di-acid chlorides, phosgene or diisocyanates [A. Lendlein and S. Kelch, Shape-memory polymers, Angew. Chem. Int Ed. 41, 2002, 2034-57 ., Ch. Min, W. Cui, J. Bei, Sh. Wang, Biodegradable shape-memory polymer-polylactide-co-poly (glycolide-co-caprolactone) Multiblock copolymer, Polym. Adv. Techn.16, 2005, 608 -15]. In these reactions, however, it is difficult to obtain copolymer of high molecular weight and low dispersion, which is a prerequisite for obtaining materials with good mechanical properties, which are required in the application of these materials in the formation of surgical implants or carriers of live cells. Also biocompatibility of products of degradation of copolymers obtained in this way is debatable, due to problems with the total purification of the product of the residues of coupling highly toxic compounds. Receipt of the required microblock structure of the SMPs copolymers without isocyanates and / or other compounds of coupling requires more than 70-80 mol%. share L-lactide in the composition (copolymers such as poly(L-lactide-co-glycolide), poly(L-lactide-co-ε-caprolactone), poly(L-lactide-co-p.dioxanone). Until the contents of more than 70% mol. lactide units in the copolymer allows for the creation of the desired crystalline phase, forming a knot in the molecular structure of the material [Min CC, WJ Cui, Bei JZ, and Wang SG, Polym. Adv. Technol., 2007, 18, 299-305] . Polymers which are obtained in this way unfortunately have a relatively low flexibility due to high participation of lactid semicrystalline phase, which greatly reduces the possibility of deformation of the material at the stage of the provisional shaping. Temperature of the return from temporary shape is also relatively high, often well above 45°C, which is associated with glass transition temperature of said copolymers and practically eliminates these copolymers from biological applications. Such materials are also characterized by a maximum stress at the time of return to the permanent shape of not more than 3.5 MPa [Xi Li Lu, W.Cai, Z. Gao, W. Tang Jun . Polymer Bulletin 58, 381-391 (2007)]. In the literature are also described copolymers containing a small proportion of the crystalline phase, of high biocompatibility, such as obtained by the terpolymerization of L-lactide, glycolide and trimethylene carbonate. Shape memory effect was obtained in these terpolymers without creating a physical network which is built of crystalline domains [Zini E., Scandola M., Dobrzynski P., Kasperczyk J., and M. Bero, Biomacromolecules, 2007, 8, 3661-3667]. Practical application of these terpolymers however bonds with a large disadvantage associated with the relatively difficult to carry out the process of shaping a temporary shape. Deformation rate must be low in this case - not higher than the rate of stretching of 15mm/min, and the temperature at which this operation is performed must be carefully stabilize and maintain close to glass transition temperature of the material. It causes that the terpolymers of this type are very troublesome in the processing and temporary shaping operations.

Surprisingly, most of these drawbacks could be eliminated by the method of preparing biodegradable, biocompatible fully polymeric materials, according to the present invention.

### Summary of invention

Bioresorbable, fully biocompatible thermoplastics, especially amorphous, which are aliphatic copolyesters or aliphatic poly(ester-co-carbonate) or poly(ester-co-amide), and poly(ester-co-carbonate-co-amide) or poly(amide-co-carbonate), which exhibit elastic properties at temperatures a little higher than the temperature of the human body in the range of 38 - 45°C, and at lower temperatures (in the range of 0° to 38°C) with a relatively high modulus of rigidity, of the controlled synthesis conditions of morphology and the shape memory properties, including long homoblocks and short mixed sequences derived from comonomers used, having the formula 7,

-(AAAA)ₙ-(ABAB)ₖ-(BBBB)ₘ- Formula 7

where n, k and m represent a natural number from 2 to 200, the component of unit A is a group of formula -O-CH(R)-CO-, -O-(CHR)_{4···6} -CO- and / or -O-(CHR)_{4···6}-O-CO-, in which R is -(CH₂)ₐR₁ or -(CHR₂ -OCH₂)_{b}-OH or -(CHR₂-OCH₂)_{b}-NH₂, where a is an integer from 0 to 10, b is an integer from 1 to 1000, R₁ is -H or -OH or -NH₂, R₂ is -CH₃ or -CH₂ CH₃, and the component of unit B is a group of formula -O-CH₂-CO-, and/or -O-(CH₂)_{4···6}-CO-, and/or -O(CH₂)_{4 ···6}-O-CO-.

Method of preparation of bioresorbable polymeric materials with shape memory according to the invention lies in the fact that the copolymerization process is carried out, extending according to a known mechanism for ROP, monomers selected from the group consisting of lactides, in particular LL-lactide and / or DD-lactide and / or L, D-lactide and / or glycolide of general formula **1** and / or lactones of general formula **2,** especially ε-caprolactone, δ-valerolactone and / or cyclic carbonates of the formula **3,** preferably trimethylene carbonate and / or dimethyl trimethylene carbonate, in the presence of aliphatic oligoestrodiols previously obtained of formula **4** with an average molecular weight Mn from1 kDa to 10 kDa and / or previously obtained aliphatic oligo(ester-co-carbonate)diols of formula **5,** having an average molecular weight Mn from 1kDa to 10 kDa, or / and oligo(ester-co-amide)diols of formula **6** with an average molecular weight Mn from 1 kDa to 10 kDa to and / or oligo(ester-co-carbonate) containing pendant hydroxyl groups and / or hydroxyethoxy ethyl of formula **8, 9** and / or propoxy propylamino of formula **10, 11** having an average molecular weight Mn from 1 kDa to 10 kDa, and specially selected low toxic initiator selected from the group consisting of zinc complexes, soluble in the monomer melt, preferably monohydrate zinc (II) acetylacetonate and / or dihydrate zinc (II) acetylacetonate and / or ethylzinc (II), and / or ethylzinc ethylate (II)) and / or zirconium complexes, soluble in the monomer melt, preferably zirconium (IV) acetylacetonate, ethoxy zirconium (IV) acetylacetonate and / or complexes of titanium (IV), preferably titanium (IV) acetylacetonate and / or complexes of iron (III), preferably iron (III) acetylacetonate.

The choice of initiator co-decides to build a chain microstructure obtained copolymer or terpolymer. Using complexes of zirconium (IV) and / or titanium (IV) and / or iron (III), which in addition to initiating the polymerization, generate strong intermolecular transesterification phenomenon, occurring in parallel to the main chain propagation reaction of the copolymer or terpolymer, and carrying out the reaction in the melt or in solution an organic solvent or mixture of organic solvents, in a relatively low temperature (in the range of 60°C to 150°C, preferably at 110°C) was obtained containing a specific mileage long homoblocks and short mixed sequences derived from comonomers used, having the formula 7,

-(AAAA)ₙ-(ABAB)ₖ-(BBBB)ₘ- Formula 7

where n, who represent a natural number from 2 to 200, the component of unit A is a group of formula -O-CH(R)-CO-, -O-(CHR)_{4···6}-CO- and / or -O-(CHR)_{4···6}-O-CO-, in which R is -(CH₂)ₐR₁ or -(CHR₂-OCH₂)_{b}-OH or -(CHR₂-OCH₂)_{b}-NH₂, where a is an integer from 0 to 10, b is an integer from 1 to 1000, R₁ is -H or -OH or -NH₂, R₂ is -CH₃ or -CH₂CH₃, and the component of unit B is a group of formula -O-CH₂-CO-, and/or -O-(CH₂)_{4 ···6}-CO-, and/or -O(CH₂)_{4 ···6}-O-CO-.

At the same time, regardless of the composition of the reaction mixture, the specificity of the trans-esterification reaction occurs involving intermolecular initiators of this group lies in the fact that their use is due to trans-esterification intermolecular course characterized by preferential attack of the active end of the growing particles of copolymer ester linkages and / or other molecules carbonate copolymer contained in the final segment blocks included in the chain of the molecule, and does not attack statistics. The result is a formation of a short sequence at the same time maintaining the composite formed in the first step of block structure reaction.

In this way are obtained amorphous and thermoplastic materials with good mechanical properties. Shape memory in obtained material is substantially associated with Contoured intermolecular interaction resulting from the presence of the intermolecular hydrogen bonds, and the specific structure of the chain, characterized by the presence of rigid segments connecting taken/busy elastic blocks.

Obtaining amorphous materials, having high elasticity and very good parameters characterizing shape memory are possible in the present invention as a result of the preparation of copolymers or terpolymers with very special segment chain microstructure containing a long elastic blocks (an average length from 10 to 200 of monomer units) connected to the shorter segments rigid, constructed with a sequence of comonomer containing pendant methyl groups and / or ethyl and / or benzyl and / or ester derived from aliphatic carboxylic acid [formula 8]. The polymerization process is carried out in the melt or solution by gradual. In the first phase the synthesis of the polymerization is carried out in the melt or in solution in an organic solvent or mixture of organic solvents (preferably: in a solution of benzene and / or chlorobenzene and / or toluene and / or tetrahydrofuran, THF) at a temperature in the range from 0°C to 200°C (preferably in toluene solution at a temperature of 50°C, in the melt at a temperature of 110°C). The reaction mixture consists of monomers such as cyclic lactones (preferably: ε-caprolactone, δ-valerolactone) and / or unsubstituted, cyclic carbonates (preferably: trimethylene carbonate) and / or derivatives of morpholine-2,5-dione (preferably: morpholino-2,5-dione, methyl morpholino-2,5-dione) and / or dicarboxylic acid anhydrides (preferably succinic acid anhydride, maleic acid anhydride).

The polymerization is carried out in the presence of a small amount of an initiator or catalyst, keeping the molar ratio of the initiator to the total moles of the comonomers I/M from 1:10000 to 1:500 (preferably from I/M = 1:2500). The initiator or catalyst in the ongoing process is a metal complex compound, preferably zinc (II) monohydroxyacetylacetonate, zirconium (IV) acetylacetonate and / or is an organic compound belonging to the group of tertiary amines or N-heterocyclic carbenes (preferably DMAP 4-dimethylaminopyridine, 4-pyrrolidinopyridine PPY) or the process of this is without a catalyst or initiator. The reaction mixture should also contain water and / or anhydrous polyhydric alcohols and / or mixtures of thereof (preferably: 1,4-butanediol and / or 1,3-propanediol and / or pentaerythritol and / or glycerin) and / or aliphatic amines, diamines and triamines primary (preferably hexane-1,6-diamine, octane-1,8-diamine, 1,4-diaminobutane, tetramethylene-1,4-diamine, guanidine) and / or aliphatic diamine or a tri- or tetra- amine secondary (preferably N,N'-dimethylhex-1,6-diamine, N,N'-dimethyloctane-1,8-diamine, spermidine N-(3-aminopropyl) -1,4-butanediamine, spermine N,N'-bis(3-aminopropyl)butane-1,4-diamine). The amount of alcohol and /or water and / or an amine determined a molecular weight of the obtained oligomer, the amount is expressed in a molar ratio of total moles of comonomers used for the total number of moles of alcohol and / or water and / or amine in the reaction mixture is in the range from 100:1 to 2500:1.

Detrimental to the further use of the materials obtained material tends to flow under constant stress at operating temperature (in this case the temperature of the human body) finally obtained amorphous copolymers or terpolymers strongly limits according to the invention by introducing the oligomer chains synthesized in the first phase of the reaction amide groups, as described above, using the cyclic derivatives of morpholine-2,5-dione in an amount of 3 mol%. to 25% mol. of the total content of the reaction mixture thus obtained and the use of an oligomeric product with 2-phase synthesis.

Tendency to flow at the operating temperature of about 37°C, and therefore the temperature of the human body eventually obtained amorphous copolymers or terpolymers, limited the present invention by placing in the first phase, during the synthesis of oligomers, pendent hydroxyl groups and / or carboxyl side-chain oligomers synthesized by use as a feedstock cyclic lactides and / or lactones and / or cyclic carbonates containing pendant hydroxyl groups and / or containing blocked chemically or / and physically hydroxyl and / or carboxyl groups. Oligomeric product so obtained is then used as the substrate in the two-phase synthesis.

Tendency to flow at the operating temperature, and thus temperature of the human body eventually obtained amorphous copolymers or terpolymers, in the present invention is limited by the synthesis of oligomers with a branched structure by using the above set of one stage of the method of polyhydric alcohols (preferably pentaerythritol and / or glycerol) and / or aliphatic amines, diamines and triamines of the primary (preferably hexane-1,6-diamine, octane-1,8-diamine, 1,4-diaminobutane, tetramethylene-1,4-diamine, guanidine), and / or aliphatic diamines or tri- or tetra- secondary amines (preferably N,N-dimethylhexan-1 6-diamine, N,N'-dimethylocta-1,8-diamine, spermidine, N-(3-aminpropyl) -1,4 -butandiamine, spermine N,N'-bis(3-aminpropyl)butan-1,4-diamine). Oligomeric product which is obtained this way in first phase, is used in a 2-phase synthesis.

### Brief description of drawings

The reaction of oligomerization is carried out to obtain the total conversion of starting materials described above, ranging from 50% to 100%. After reaching a conversion of the objective reaction mixture, an melt or a solution obtained in the first stage of synthesis of the products in order to obtain copolymers or terpolymers, according to the invention lactides are added (glycolide preferably, LL-lactide, L,D-lactide, DD-lactide) and / or lactones containing side groups (preferably 5,5-dimethyloxepan-2-one, 6-(benzyloxy)oxepan-2-one, benzyl(7-oxooxepan-3-yl)-acetonate) and / or cyclic carbonate containing side groups (preferably 5,5-dimethyl-1,3-dioxan-2-one, 5-(benzyloxy)-1,3-dioxan-2-one). After melting or dissolution of the cyclic monomers to the reaction mixture is added the copolymerization initiator, and started second phase of synthesis, which is carried out at a temperature from 60°C to 200°C (preferably in the temperature range from 80°C to 120°C). As the initiator of this phase of the reaction is used alkoxide, salts and complexes of zirconium (IV) and / or titanium (IV) (preferably zirconium (IV) acetylacetonate, anhydrous six chloride zirconium (IV), t-butoxy zirconium (IV), titanium acetylacetonate (IV)), which in addition to the effective capacity of the initiating ROP reaction, responsible for the creation of a high-molecular copolymer or terpolymer, cause the phenomenon of strong intermolecular transesterification, resulting in the formation of the chain structure containing strongly mixed and relatively short segments consisting of derivatives of 2-phase added substrates. In carrying out the copolymerization in the moderate temperatures in the range from 50°C to 130°C, transesterification due to the presence of the said groups of initiators, runs specifically. In these conditions occurs the transesterification reaction or intermolecular transcarbonization, this reaction is largely redirected to the final ester and / or carbonates bonds of blocks present in the chain. Such intermolecular transesterification mechanism causes a relatively slow and small decrease in block length from the long oligomer molecules present in the reaction mixture, formed in parallel with a large number of short-chain segments consisting of mixed sequence, derived from the reaction medium entering the cyclic monomers.

In copolymerization or terpolymerization which is carried in 2-phase, catalyst or initiator is not added, if used in the first stage initiators or catalysts such as zinc compounds (preferably monohydrate zinc (II) acetylacetonate) and / or compounds of zirconium (IV) (preferably zirconium (IV) acetylacetonate) or compounds of titanium (IV) (preferably butoxide titanium (IV)).

In the case of application in phase 1 and / or 2 cyclic monomers containing side groups low reactive, but which can be activated by a chemical reaction to the active hydroxyl and / or carboxylic groups, the reaction is carried out to obtain activation of active hydroxyl and / or carboxylic groups in the copolymer or terpolymer chain after the end of the 2 phase of reaction and the separation and purification of the product by reaction with hydrogen in the presence of a platinum catalyst (preferably, a reaction of reduction benzyloxy and / or phenylacetate groups). The presence of active hydroxyl and / or carboxylic groups in the chain of the copolymer or terpolymer increases the strength of intermolecular interactions, resulting in a decrease in the tendency to mainly flow of this material, an increase in mechanical strength and improvement of the parameters characterizing the shape memory properties, such as degree of return to the permanent form, the rate of return, whether the value of the stress released in time to reverse the phenomenon of permanent shape.

Method of preparation of to bioresorbable, fully biocompatible thermoplastic, especially amorphous, which are aliphatic copolyesters or aliphatic poly(ester-co-carbonate) or poly(ester-co-amide), and poly(ester-co-carbonate-co-amide), or poly(amide-co-carbonate), which exhibit elastic properties at temperatures a little higher than the temperature of the human body in the range of 38° - 45°C, and at lower temperatures (in the range of 0° to 38°C) with a relatively high modulus of rigidity, of the controlled synthesis conditions of morphology and the shape memory properties. The polymeric material obtained by the invention does not contain toxic impurities, because all substrates used in the synthesis and the obtained products are biocompatible. It does not contain even traces of toxic compounds such as isocyanates, acid chlorides, acrylic monomers, toxic amines, tin compounds, etc., which are present in such products obtained by the reaction of the previously described and whose complete removal is very troublesome and expensive. Initiators and / or catalysts used in the method have been specially selected to ensure full biocompatibility of the products obtained.

Thermoplastics, obtained according to the invention, are programmed at synthesis stage properties, through specific selection of the composition of the substrate, selection of the type of initiator or catalyst used, selection of appropriate methods and conditions of the copolymerization. All of these factors shape the final sectional structure of synthesized chain of the copolymer or terpolymer, which is characterized by the presence of both long flexible monomer units and short rigid chain segments composed of mixed comonomer sequences. This structure allows to obtain a copolymer or terpolymer having a shape memory effect, regardless of the degree of crystallinity of the material intended to form temporary surgical implants, especially for use in minimally invasive surgery and / or as a material in the production of drug carriers for the controlled release and / or for the formation of three-dimensional substrates for the cultivation of cells and tissues that can respond to a thermal stimulus by controlled change of mechanical properties and shape. These materials, due to the high yield strength at temperatures above 38°C, and the phenomenon of strong intermolecular interaction afforded by the special shape of a structure of the copolymer chain and the appropriate selection of the composition is also characterized by ease of shaping a temporary shape and relatively high speed to return to the permanent shape after thermal activation stimulus - temperature close to human body temperature (36-41°C). The method of the invention can greatly reduce the typical tends of this type of material to flow phenomenon of the polymer material at a temperature of the human body. The method of the present invention allows to obtain amorphous polymers with very favorable profile of hydrolytic degradation, particularly useful in the process of the controlled release of drugs, having a good shape memory properties.

Process for the preparation of polymeric materials with shape memory, which is the subject of the invention, in contrast to previously known methods for the preparation of such materials provides the resulting products, while maintaining the shape memory phenomenon, a high biocompatibility of the material, and full biocompatibility of products formed during the degradation of the material, and during the release of the support compounds in the process used for the synthesis. Material obtainable according to the invention exhibits complete biodegradability, relatively high modulus at temperatures below 38°C, thermoplasticity and good processing properties allow processing by injection molding, extrusion, pressing, ease of programming the temporary shape which involves the high plasticity of the material already at temperatures of 39 - 45°C and durability of strong intermolecular interactions forming physical "nodes" at temperature of run deformation. Prepared in accordance with the invention, a polymeric material, characterized by a set of these properties is therefore especially valuable for biomedical applications.

The end result of the invention are copolymers or terpolymers with sectional structure of the chain, comprising a flexible blocks of a size between 10 to 200 monomer units, joined with rigid shorter sequences [Formula 1]. These blocks in the copolymer chain, thanks to its flexibility, giving to the received materials the ability for easy of their processing, their controlled deformation during temporary shaping. By appropriate selection of reactants in the first stage of this method of synthesis, it is possible to adjust the final flexibility and other thermo-mechanical parameters of derived polymeric materials. These sequences due to the chemical structure and presence of steric hindrance act as a rigid physical network nodes. The observed phenomenon of "physical crosslinking" is increased by the presence of molecular interactions associated with the presence of introduced polar side groups in the rigid segments of chain. By selecting the type and amount of polar side groups in the rigid segments of chain it is possible to, in accordance with the invention, modeling thermomechanical properties of obtained polymer materials.

According to the invention, by selecting the type of initiator and / or catalyst, using the same monomers can also change the chain microstructure of the obtained copolymers and their morphology, by placing the crystalline domains. In applying the compounds of zinc (II) as a reaction initiator (preferably monohydrate zinc (II) acetylacetonate, difenylzinc (II), ethyletoxy zinc (II), diethylzinc (II)) and performing the reaction in the melt or solution of an organic solvent or mixture of organic solvents, in accordance with the invention, a relatively low temperature (in the range of 50°C to 200°C, preferably at a temperature of 100°C), causes the inhibition or serious limitation of the transesterification reaction in the construction of the final chains structure of obtained copolymers or terpolymers. As a result of this phenomenon resulting chain structure of the copolymer or terpolymer is a multiblock, a large proportion of long and medium long monoblocks. Thermoplastic materials, semi-crystalline, with good mechanical properties and a relatively high modulus are obtained. Memory shape in materials obtained in this way is linked to the both the existence of crystalline domains resulting microblocks separation as well as with the presence of intermolecular hydrogen bonding. By appropriate choice of initiator and / or catalyst and / or catalyst system which is a mixture of compounds mentioned above, and the selection of reaction temperature and / or temperature changes in the reaction carried out during the process, can be controlled easily the degree of crystallinity of obtained polymeric materials, and thus modify the final property.

In order to intensify the interactions of the chains of obtained copolymers or terpolymers, and obtain a chain structure containing long rigid blocks, which allows the formation of crystalline domains in the polymer material produced, by the method of the invention, the composition of the reaction mixture in addition to the cyclic lactides and / or lactones, and / or carbonates consisting terminated with active group or hydroxyl groups, consisting chain stiffening side groups oligoesters aliphatic (preferably oligo(LL-lactide), oligo(DD-lactide), oligo(DD-lactide-co-LL-lactide, oligo(LL-lactide-co-glycolide), oligo(DD-lactide-co-glycolide) and / or aliphatic oligocarbonatetes containing side groups (preferably oligo(5,5-dimethyl-1,3-dioxan-2-one) and / or aliphatic oligo(ester-co-carbonates), preferably oligo(3,6-dimethyl-1,4-dioxane-2,5-dione-co-5,5-dimethyl-1,3-dioxan-2-one ) and / or aliphatic oligo(ester-co-amide), preferably oligo(3,6-dimethyl-1,4-dioxane-2,5-dione-co-3,6-dimetylomorpholino-2,5-di one, oligo(3,6-dimethyl-1,4-dioxane-2,5-dione-co-L-valine).

In order to intensify the molecular interactions of the chains of obtained copolymers or terpolymers, and obtain a structure containing a long rigid blocks, which enables formation of crystalline domains in the polymer material produced by the method of the invention, the process is carried out in two stages. In the first phase of the reaction by ROP reaction of cyclic lactides with side groups (preferably LL-lactide, DD-lactide) and / or lactones containing side groups (preferably, dimetylooxepan-2-one, 6-(benzyloxy)oxazepam-2-one) and / or derivatives of morpholine (preferably 3,6-dimethylomorpholino-2,5-dione) carried out in a solution of organic solvent (preferably, tetrahydrofuran, toluene, chloroform) or a mixture of organic solvents (preferably: a solution of chlorobenzene and /or toluene and / or and tetrahydrofuran), or in a melt at a temperature in the range from 0°C to 200°C (preferably in toluene solution at a temperature of 50°C, in the melt at a temperature of 110°C) gave the oligoesters and / or oligoestrocarbonates and / or oligoestroamids. The reaction is carried out in the presence of a small amount of an initiator or catalyst, keeping the molar ratio of the initiator to the total moles of the comonomers I / M from 1:10000 to 1:500 (preferably from I / M = 1:2500). The initiator or catalyst in the ongoing process is a metal complex compound (preferably zinc (II) monohydroxyacetylacetonate, zirconium (IV) acetylacetonate) and / or an organic compound belonging to the group of tertiary amines or N-heterocyclic carbenes (preferably DMAP 4-dimethylaminopyridine, 4-pyrrolidinopyridine PPY), or the process is carried without catalyst or initiator. The reaction mixture should also contain water and / or aqueous polyhydric alcohols and / or mixtures thereof (preferably: 1,4-butanediol and / or 1,3-propanediol and / or pentaerythritol and / or glycerin) and / or aliphatic primary amines and diamines (preferably hexane-1,6-diamine, octane-1,8-diamine) and / or secondary aliphatic diamine (preferably N,N'-dimethylhexane-1,6-diamine, N,N'-dimethyloctane-1,8-diamine). The amount of alcohol or water determines the molecular weight of the resulting oligomer, the amount is expressed in a molar ratio of the sum of moles of comonomer to the molar amount of alcohol in the reaction mixture is in the range of from 100:1 to 2000:1. After obtaining the conversion of the substrates (50 to 100%), (preferably 98%) by the method according to the invention, starts with the second phase of the synthesis reaction by adding to the mixture of products obtained in the first stage of the monomers such as cyclic lactones (preferably: ε-caprolactone, δ-valerolactone) and / or glycolide and / or unsubstituted, cyclic carbonates (preferably: trimethylene carbonate) and / or cyclic derivatives of morpholine-2,5-dione (preferably: 3-methylmorpholine-2,5-dione, 6-methylmorpholine-2,5-dione). After melting the cyclic monomer or dissolved in a solution, to the reaction mixture is added the copolymerization initiator, which starts the second phase of synthesis, which is carried out at a temperature from 60°C to 180 °C (preferably in the temperature range from 80°C to 120°C). As an initiator of this phase of the reaction is used alkoxide, salts and complexes of zinc (II) (preferably, zinc (II) acetyacetonate monohydrate and anhydrous zinc (II) dichloride, phenylzinc (II) and / or ethylzinc (II), and / or ethoxy ethylzinc ( II). Using as an initiator of said compounds of zinc (II), results in a limitation of the transesterification reaction in the construction of the final structure of the chains of obtained copolymers or terpolymers. As a result of this phenomenon, resulting chain structure of the copolymer or terpolymer is a block (with a block of oligomers obtained in the first phase of described reaction). In this way the method according to the invention is obtained thermoplastic materials, semi-crystalline, with good mechanical properties and a sufficiently high modulus. Shape memory in materials obtained in this way is substantially related to the presence of crystalline domains, formed by the separation of rigid long chain fragments. Flexible amorphous phase, which plasticizing the final copolymer or terpolymer, formed in so obtained materials the sequences and the blocks formed by polymerization of the monomers added to the reaction mixture in the second stage of the synthesis.

As the initiator of this phase are used alkoxides, salts and complexes of zirconium (IV) and / or titanium (IV) (preferably, zirconium (IV) acetylacetonate, anhydrous zirconium (IV) chloride, t-butoxy zirconium (IV), titanium (IV) acetylacetonate, while keeping the reaction temperature to 140°C. The use of those complexes of zirconium (IV) or titanium (IV) in addition to initiating the copolymerization process causes a specific reaction of transesterification or transcarbonization, which resulting in the creation of the sectional structure of the chain, comprising both long rigid blocks stored in the first phase resulting from the reaction as described and mixed short sequences , irrespective of the composition of the reaction mixture, essentially derived from the monomers added in the second stage of the reaction, carrying out the reaction in the melt or in solution in an organic solvent or in mixture of organic solvents, at a relatively low temperature (in the range of 60°C to 140°C, preferably at 110°C). Thermoplastic materials, semi-crystalline, with good mechanical properties and high modulus of rigidity are obtained. Shape memory in obtained in this way materials is associated with the presence of crystalline domains, formed by the segregation of the rigid long chain fragments. Flexible amorphous phase, which plasticizing the final copolymer or terpolymer formed in so obtained materials the sequences and microblocks formed by polymerization of the monomers added to the reaction mixture in the second stage of the synthesis. As a result of running specific reaction of transesterification and / or transcarbonization, some part of these sequences also include groups derived from monomers containing side groups, applied in the first phase of the reaction, thus reducing the susceptibility of the final material to flow at temperatures close to the glass transition temperature of the resulting polymeric material.

### Description of embodiments

The following are examples of preparation according to the invention, the bioresorbable thermoplastic materials having a shape memory, in no way limiting to claim previously demonstrated:

Example I

Bioresorbable material was obtained, showing the shape memory through a two-step reaction.

Step-1

A reactor with a capacity of 50 cm³, connected to a vacuum line and with a supply of dried argon mixture was introduced 10.2 g (0.1 mol) of trimethylene carbonate (TMC) with 0.23 g (2.5*10⁻³ mol) of 1,4-butanediol and 0.01 g of monohydrate zinc (II) acetylacetonate. The reactor contents were melted, and then degassed. The obtained melt was heated in a closed reactor under a blanket of argon for 3 hours at 120°C.

To give a mixture of 10 g of oligo(trimethylocarbonate) terminated with hydroxyl groups with a number average molecular weight 4 000 g/mol, a glass transition temperature of -15°C and about 0.2 g of unreacted trimethylocarbonate. The product obtained, without further purification procedures were used to carry out the second stage of the synthesis.

Step-2

In a reactor with a capacity of 100 cm³, connected to a vacuum line and with a supply of dried argon 3.06 g were melted the mixture of oligo(trimethylocarbonate) obtained before and trimethylocarbonate. Subsequently the reactor was charged with 15.12 g (0.105 mol) of L-lactide, 1.74 g (0.015 mol) of glycolide, and 0.0488 g zirconium (IV) acetylacetonate. The content of the reactor was degassed and stirred in a closed reactor under a blanket of argon at 120°C for 120 hours. The product was purified from the residue of monomers by dissolving in chloroform and precipitated with cold methanol, and then dried under vacuum to constant weight.

To give 19 g of amorphous terpolymer of number average molecular weight of 34 000 g / mol, the molecular weight dispersity Mw / Mn = 1.8, glass transition temperature Tg = 41°C, flexural strength, maximum elongation, and Young's modulus, respectively;

at 25°C, 58.4 MPa, 4.8% and 2200 MPa,

at 37°C, 33 MPa, 10.1 % and 1350 MPa,

at 42°C, 2.7 MPa, more than 200% and 360 MPa

From the obtained material by injection, rectangular moulder was made with dimensions of 10 mm x 5 mm x 50 mm (permanent shape), and then at 45°C it was stretched and then rapidly cooling to room temperature, it was given a temporary shape with a length of 100 mm (100% elongation). After placing the temporary shaped moulder in a water bath at 43°C was observed automatic return to a shape permanent in time 34 s. After cooling to room temperature, degree of return to the permanent shape was 98%.

Example II

Bioresorbable material was obtained, showing the shape memory through a two-step reaction.

Step-1

A reactor with a capacity of 50 cm³, connected to a vacuum line and with a supply of dried argon mixture was introduced 10.2 g (0.1 mol) of trimethylenecarbonate (TMC) with 0.18 g (1.5*10⁻³ mol) of 1, 6-hexanediol monohydrate and 0.01 g of zinc (II) acetylacetonate. The reactor contents were melted, and then degassed. The obtained melt was heated in a closed reactor under a blanket of argon for 3 hours at 120°C.

To afford 10 g of oligo(trimethylenecarbonate) terminated with hydroxyl groups with a number average molecular weight of 8 000 g / mol, a glass transition temperature of -13°C. The product was used without further purification, as a substrate to carry out the second stage of the synthesis.

Step-2

In a reactor with a capacity of 100 cm³, connected to a vacuum line and with a supply of dried argon 3.06 g of oligo(trimethylenecarbonate) were melted, which were obtained in the first step. Subsequently the reactor was charged with 15.12 g (0.105 mol) of L-lactide, 1.74 g (0.015 mol) of glycolide. Not applied any additional initiator. The content of the reactor was degassed and stirred in a closed reactor under a blanket of argon at 190°C for 10 hours. The product was dissolved in chloroform and precipitated with cold methanol, and then dried under vacuum to constant weight.

To give 19 g of amorphous terpolymer of number average molecular weight of 39 000 g / mol, the molecular weight dispersity Mw / Mn = 2.2 and a glass transition temperature of 39 °C.

flexural strength, maximum elongation, and Young's modulus, respectively;

at 25°C, 48.4 MPa, 6.1 % and 2000 Mpa,

at 37°C, 29 MPa, 12.2% and 1100 MPa,

at 42°C, 1.8 MPa, more than 200% and 100 MPa

From the obtained material by injection, rectangular moulder was made with dimensions of 10 mm x 5 mm x 50 mm (permanent shape), and then at 45°C it was stretched and then rapidly cooling to room temperature, it was given a temporary shape with a length of 100 mm (100% elongation). After placing the temporary shaped moulder in a water bath at 43°C was observed automatic return to a shape permanent in time 25 s. After cooling to room temperature, degree of return to the permanent shape was 96%.

EXAMPLE III

Bioresorbable material was obtained, showing the shape memory through a two-step reaction.

Step-1

A reactor with a capacity of 50 cm³, connected to a vacuum line and with a dried argon inlet were introduced 8.16 g (0.08 mol) of trimethylene carbonate (TMC), 2.28 g (0.02 mol) ε-caprolactone and 0.14 g (1.0*10⁻³ mol) of pentaerythritol and 0.03 g of 20% sodium ethoxide ethylzinc (II) in tetrahydrofuran. The reactor contents were melted in temperature 100°C, and then degassed. The obtained melt was heated in a closed reactor under a blanket of argon for 1 hour at 110°C.

To afford 10 g of oligo(trimethylocarbonate) terminated with hydroxyl groups with a number average molecular weight of 11 000 g / mol, a glass transition temperature of -18°C. The product was used to carry out the second stage of the synthesis.

Step-2

In a reactor with a capacity of 100 cm³, connected to a vacuum line and with a supply of dried argon 3.06 g of oligo(trimetylenocarbonate-co-ε-caprolactone) were melted, which were obtained in the first step. Subsequently the reactor was charged with 15.12 g (0.105 mol) of L-lactide, 1.74 g (0.015 mol) of glycolide and 0.02 g of iron (III) acetylacetonate. The content of the reactor was degassed and stirred in a closed reactor under a blanket of argon at 120°C for 12 hours. The product was dissolved in chloroform and precipitated with cold methanol, and then dried under vacuum to constant weight.

To give 16.3 g of amorphous terpolymer of number average molecular weight of 51 000 g / mol, the molecular weight dispersity Mw / Mn = 2.2 and a glass transition temperature of 37°C.

flexural strength, maximum elongation, and Young's modulus, respectively;

at 25°C, 38.4 MPa, 7.1 % and 1900 MPa,

at 37°C, 19.1 MPa, 20.4% and 1000 MPa,

at 42°C, 1.2 MPa, 200% and 80 Mpa

From the obtained material by injection, rectangular moulder was made with dimensions of 10 mm x 5 mm x 50 mm (permanent shape), and then at 42°C it was stretched and then rapidly cooling to room temperature, it was given a temporary shape with a length of 100 mm (100% elongation). After placing the temporary shaped moulder in a water bath at 41°C was observed automatic return to a shape permanent in time 19 s. After cooling to room temperature, degree of return to the permanent shape was 98%.

Example IV

Bioresorbable material was obtained, showing the shape memory through a one-step reaction.

In a reactor with a capacity of 100 cm³, connected to a vacuum line and with a dried argon inlet were introduced 15.12 g (0.105 mol) of L,L-lactide. After it melted at 150°C, the contents of the reactor was degassed and stirred under a blanket of argon, followed by addition of 0.05 g of t-butoxy zirconium (IV). While maintaining the temperature of lactide homopolymerization was carried out for a further 30 minutes. After this time the reaction mixture, with vigorous stirring, was gradually added 2.08 g (0.018 mol) of glycolide and 2.75 g (0.027 mol) of trimethylene carbonate, by lowering the temperature of the reaction mixture to 120°C. Reactions were carried out at this temperature for a further 70 hours. The product was dissolved in chloroform and precipitated with cold methanol, and then dried under vacuum to constant weight.

To give 19.1 g of semicrystalline terpolymer of number average molecular weight of 69 000 g / mol, the molecular weight dispersity Mw / Mn = 2.0, glass transition temperature Tg = 41°C, a melting point of the crystalline phase Tm = 140°C, heat of crystallization dHc = 23 J / g and heat of fusion of the crystalline phase dHm 19 J / g.

flexural strength, maximum elongation, and Young's modulus, respectively;

at 25°C, 45.3 MPa, 2.1% and 2200 MPa,

at 37°C, 29.0 MPa, 3.8% and 1500 MPa,

at 42°C, 9.2 MPa, 79% and 470 MPa

From the obtained material by injection, rectangular moulder was made with dimensions of 10 mm x 5 mm x 50 mm (permanent shape), and then at 42°C it was stretched and then rapidly cooling to room temperature, it was given a temporary shape with a length of 75 mm (50% elongation). After placing the temporary shaped moulder in a water bath at 44°C was observed automatic return to a shape permanent in time 29 s. After cooling to room temperature, degree of return to the permanent shape was 95%.

Example V.

Bioresorbable material was obtained, showing the shape memory through a two-step reaction.

Step-1, obtaining macrodiols, oligo(butyl succinate).

A reactor with a capacity of 250 cm³, connected to a vacuum line and with a dried argon inlet, stirrer, condenser, thermometer, and collecting the distillate mixture was introduced 25.1 g (0.29 mol) of 1,4-butanediol with 33.6 g (0.23 mol) dimethyl succinate and 0.1 g of tetra-n-butoxide titanium (IV). The reactor contents were degassed and heated in an oil bath. The reaction temperature rose to 150°C for 20 minutes. The temperature was then gradually raised to 10°C per minute to 200°C. Distilled methanol which was formed during the reaction. Subsequently the reaction was carried out under reduced pressure (<1.5 mm Hg) and under these conditions the reaction mixture was maintained for 2.5 hours.

To give 8.8 g of oligo(butyl succinate) having a number average molecular weight 1 000 g / mol, a glass transition temperature of -27°C. The product was used without further purification, as a substrate to carry out the second stage of the synthesis.

Step-2, the synthesis of a terpolymer with a shape memory containing the crystalline phase

In a reactor with a capacity of 100 cm³, connected to a vacuum line and with a supply of dried argon and stirring 2.88 g melted oligo(butyl succinate) obtained in the first step. Subsequently the reactor was charged with 14.4 g (0.1 mol) of L-lactide, 1.62 (0.014) g of glycolide and 0.061 g of zirconium (IV) acetylacetonate. The content of the reactor was degassed and stirred in a closed reactor under a blanket of argon at 120°C for 72 hours. The product was dissolved in chloroform and precipitated with cold methanol, and then dried under vacuum to constant weight.

To give 18 g of crystalline terpolymer of number average molecular weight of 20 000 g / mol, the molecular weight dispersity Mw / Mn = 2.8 and a glass transition temperature of 37°C.

flexural strength, maximum elongation, and Young's modulus, respectively;

at 25°C, 31.2 MPa, 4.2 % and 1900 MPa,

at 37°C, 27 MPa, 6.1 % and 1200 MPa,

at 42°C, 19 MPa, more than 92% and 800 MPa

From the obtained material by injection, rectangular moulder was made with dimensions of 10 mm x 5 mm x 50 mm (permanent shape), and then at 45°C it was stretched and then rapidly cooling to room temperature, it was given a temporary shape with a length of 100 mm (100% elongation). After placing the temporary shaped moulder in a water bath at 41°C was observed automatic return to a shape permanent in time 240 s. After cooling to room temperature, degree of return to the permanent shape was 80%.

Example VI.

Bioresorbable material was obtained, showing the shape memory through a two-step reaction.

Step-1 Preparation of hydroxyl and / or amino groups terminated oligoesteramide

A reactor with a capacity of 250 cm³, connected to a vacuum line and with a dried argon inlet, stirrer, condenser, thermometer, and collecting the distillate mixture was introduced 25.1 g (0.29 mol) of 1,4-butanediol with 33.6 g (0.23 mol) dimethyl succinate, 13.59 g (0.115 mol) of hexane-1,6-diamine and 0.1 g of tetra-n-butoxide titanium (IV). The reactor contents were degassed and heated in an oil bath. The reaction temperature rose to 150°C for 20 minutes. The temperature was then gradually raised to 10°C per minute to 200°C. Distilled methanol which was formed during the reaction. Subsequently the reaction was carried out under reduced pressure (<1.5 mm Hg) and under these conditions the reaction mixture was maintained for 2.5 hours.

To give 10.5 g of oligoesteramide number average molecular weight 1 000 g / mol, as determined by NMR analysis of the terminal groups and a molecular weight of 1150 g / mol, determined on the basis of number of -OH groups and / or -NH₂. Glass transition temperature was set -35°C. The product was used without further purification, as a substrate to carry out the second stage of the synthesis.

Step-2, to obtain a copolymer with shape memory, with a low flow of the temperature close to body temperature

In a reactor with a capacity of 100 cm³, connected to a vacuum line and with a supply of dried argon and stirring, molten 4.69 g of oligoestroamide obtained in the first step. Subsequently the reactor was charged with 14.4 g (0.1 mol) of L-lactide and 0.061 g zirconium (IV) acetylacetonate. The content of the reactor was degassed and stirred in a closed reactor under a blanket of argon at 120°C for 72 hours. The product was dissolved in chloroform and precipitated with cold methanol, and then dried under vacuum to constant weight.

To give 19 g of semicrystalline polymer with a number average molecular weight of 19 000 g / mol (determined on the basis of GPC using a styrene standard), the molecular weight dispersity Mw / Mn = 2.9 and a glass transition temperature of 36°C.

Flexural strength, maximum elongation, and Young's modulus, respectively;

at 25°C, 30 MPa, 4% and 1700 MPa,

at 37°C, 28.2 MPa, 10.9% and 1100 MPa,

at 42°C, 20 MPa, more than 65%, and 700 MPa

From the obtained material by injection, rectangular moulder was made with dimensions of 10 mm x 5 mm x 50 mm (permanent shape), and then at 45°C it was stretched and then rapidly cooling to room temperature, it was given a temporary shape with a length of 100 mm (100% elongation). After placing the temporary shaped moulder in a water bath at 40°C was observed automatic return to a shape permanent in time 200 s. After cooling to room temperature, degree of return to the permanent shape was 82%.

Example VII.

Bioresorbable material was obtained, showing the shape memory through a two-step reaction.

Step-1 to obtain a monomer, 5-benzyloxy-1,3-dioxan-2-one and its oligomerization to makrodiols

A reactor with a capacity of 100 cm³ is connected to a vacuum line and with a dry nitrogen inlet, and a stirrer were introduced a mixture of 12.74 g (0.07 mol) of 5-benzyloxy-1,3-propanediol, 23.7 ml (0.245 mol) of ethyl chloroformate and 265 ml of distilled tetrahydrofuran. The solution was stirred at 0°C under a nitrogen blanket for 15 minutes. At the time of 30 minutes was added dropwise 32.5 ml (0.23 mol) of triethylamine. The reactor contents were stirred at a temperature of 0°C to room temperature over 3 hours. The resulting precipitate was filtered off, the solvent was evaporated. The reaction yield is 67%. The residue was recrystallized from a process of THF / diethyl ether.

The resulting 5-benzyloxy-1,3-dioxan-2-one, in an amount of 20 g (0.1 m), and 0.2 g of 1,4-butanediol in a reactor provided with a capacity of 50 cm³ is connected to a vacuum line and with a dry nitrogen inlet. The reaction vessel is placed in an oil bath heated to 140°C. After five minutes, from the melt of the monomer, initiator introduced - zinc (II) octoate (0.07 g 0.1 mol. solution in anhydrous THF). The reaction was carried out for 4 hours, and then the resulting mixture was cooled to room temperature within two hours. The product was purified by dissolving in methylene chloride and precipitated with cold methanol, and then dried under vacuum to constant weight.

To give 15.2 g oligocarbonate, a number average molecular weight determined by gel permeation chromatography GPC, equal to 4700 g /mol, from the observation of end groups by NMR equal to 8500 g / mol and based on the determination of the -OH groups of about 9000 g / mol.

Step-2 Obtaining of shape memory polymer, with increased hydrophilicity and a low flow of body temperature

In a reactor with a capacity of 100 cm³, connected to a vacuum line and with a supply of dried introduced 7.14 g of oligocarbonate obtained in the first step. Subsequently the reactor was charged with 15.84 g (0.11 mol) of L-lactide, 2.32 g (0.02 mol) of glycolide and 0.065 g of zirconium (IV) acetylacetonate. The content of the reactor was degassed and stirred in a closed reactor under a blanket of argon at 120°C for 72 hours. The product was dissolved in chloroform and precipitated with cold methanol, and then dried under vacuum to constant weight.

The dried product was subjected to hydrogenation process, 20 g of polymer was dissolved in 150 ml of tetrahydrofuran, then was placed in a hydrogenation vessel, was added palladium catalyst - 3.3 g 10% Pd / C and 3.3 g Pd(OH)₂ / C. The content of the vessel was degassed and H₂ gas was passed through twice. The reaction mixture was stirred for 24 hours in a high pressure (4 bar). Subsequently the reaction mixture was filtered using celite adsorbent. The resulting precipitate was washed with 250 ml of THF, and then dried under vacuum to constant weight.

18 g of a polymer containing hydroxyl groups, a number average molecular weight of 21 000 g / mol (determined on the basis of GPC using a styrene standard), the molecular weight dispersity Mw / Mn = 2.8 and a glass transition temperature of 47°C.

Flexural strength, maximum elongation, and Young's modulus, respectively;

at 25°C, 33 MPa, 4.8% and 1900 MPa,

at 37°C, 27 MPa, 8.8% and 1300 MPa,

at 42°C, 21 MPa, more than 70% and 800 MPa

From the obtained material by injection, rectangular moulder was made with dimensions of 10 mm x 5 mm x 50 mm (permanent shape), and then at 45°C it was stretched and then rapidly cooling to room temperature, it was given a temporary shape with a length of 100 mm (100% elongation). After placing the temporary shaped moulder in a water bath at 50°C was observed automatic return to a shape permanent in time 180 s. After cooling to room temperature, degree of return to the permanent shape was 88%.

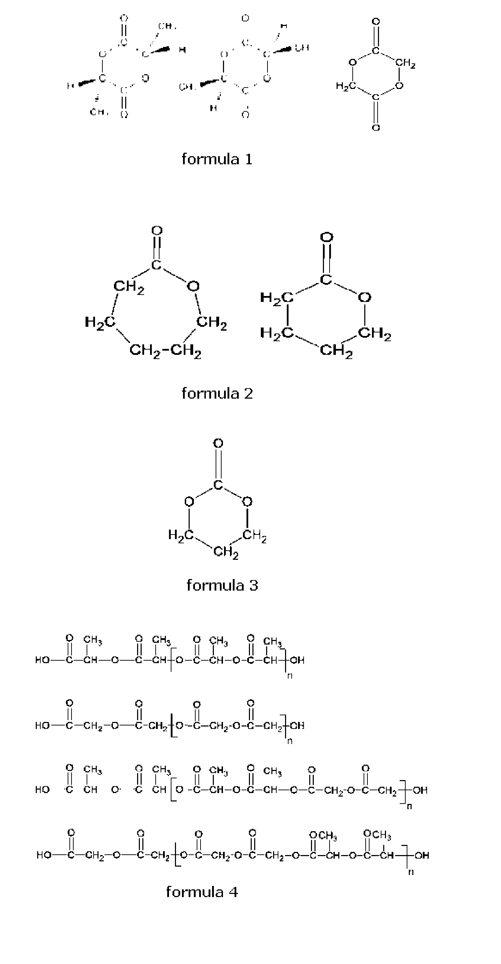

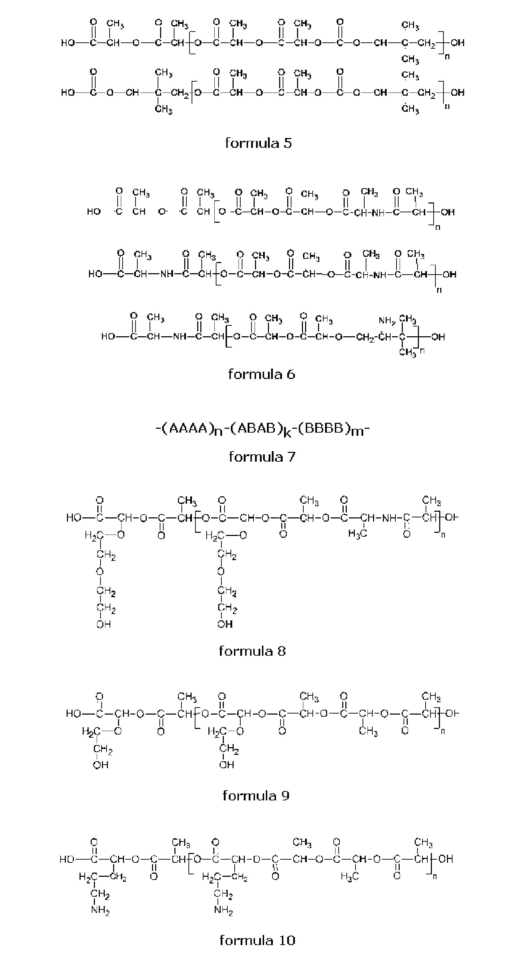

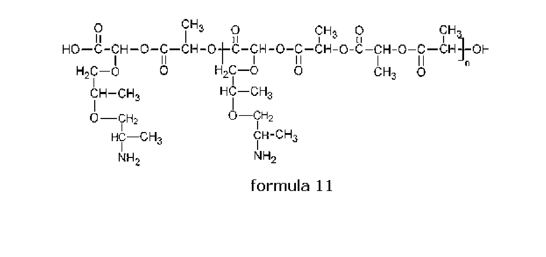

## Claims

1. Bioresorbable, fully biocompatible thermoplastics, especially amorphous, which are aliphatic copolyesters or aliphatic poly(ester-co-carbonate) or poly(ester-co-amide), and poly(ester-co-carbonate-co-amide) or poly(amide-co-carbonate), with the participation of the ester units of 20 to 80 mol%, carbonate of 20 to 80 mol%, and the amide of 1 to 30 mol% of exhibiting elastic properties at temperatures a little higher than the temperature of the human body in the range of 38 - 45°C, and at lower temperatures (in the range of 0° to 38°C) with a relatively high modulus of rigidity, of the controlled synthesis conditions of morphology and the shape memory properties, including long homoblocks and short mixed sequences derived from comonomers used, having the formula 7: -(AAAA)ₙ-(ABAB)ₖ-(BBBB)ₘ- where n, k and m represent a natural number from 2 to 200, the component of unit A is a group of formula -O-CH(R)-CO-, -O-(CHR)_{4···6}-CO- and / or -O-(CHR)_{4···6} -O-CO-, in which R is -(CH₂)ₐR₁ or -(CHR₂-OCH₂)_{b}-OH or -(CHR₂-OCH₂)_{b} -NH₂, where a is an integer from 0 to 10, b is an integer from 1 to 1000, R₁ is -H or -OH or -NH₂, R₂ is -CH₃ or -CH₂CH₃, and the component of unit B is a group of formula -O-CH₂-CO-, and/or -O-(CH₂)_{4···6}-CO-, and/or -O(CH₂)_{4 ···6} -O-CO- having a high modulus at temperatures below 38°C and exhibiting elastic properties at temperatures a little higher than the temperature of the human body is in the range 38-45°C, have a high ease of programming the temporary shape, morphology-controlled synthesis conditions and parameters of the shape memory properties.

2. The method for the preparation of biocompatible and bioresorbable thermoplastic elastomer having a shape memory, particularly for biomedical applications in the process of copolymerization, running in accordance with the known mechanism of ROP, wherein the copolymerization is carried out, the monomers selected from the group consisting of lactides, in particular LL-lactide and / or DD- lactide and / or L,D-lactide and / or glycolide of general formula 1 and / or lactones of general formula 2, especially ε-caprolactone, δ-valerolactone and / or cyclic carbonates of the formula 3, preferably trimethylene carbonate and / or dimethyl trimethylene carbonate, in the presence of aliphatic oligoestrodiols previously obtained of formula 4, with an average molecular weight Mn of 1 kDa to 10 kDa and / or previously obtained aliphatic oligo(ester-co-carbonate)diol of formula 5, having an average molecular weight Mn of 1kDa to 10 kDa and / or oligo(ester-co-amide)diols of formula 6 with an average molecular weight Mn of 1 kDa to 10 kDa and specially selected low toxic initiator selected from the group consisting of complexes of soluble zinc in the alloy of the monomers, preferably monohydrate zinc (II) acetylacetonate and / or dihydrate zinc (II) acetylacetonate and / or ethylzinc (II), and / or ethylzinc (II) ethoxide and / or complexes of zirconium soluble in the alloy of monomers, preferably zirconium (IV) acetylacetonate, zirconium (IV) ethoxy acetylacetonate and / or complexes of titanium (IV), preferably titanium (IV) acetylacetonate and / or complexes of iron (III), preferably iron (III) acetylacetonate.

3. The method according to claim. 2, **characterized in that** the reaction is carried out in the melt or in solution in an organic solvent or mixture of organic solvents, at a relatively low temperature in the range of 60°C to 150°C, preferably at 110°C, to obtain a specific course of transesterification, which results in resulting in formation of the segment structure of the chain, containing the long homoblocks and short mixed sequences derived from comonomers used.

4. The method according to claim. 2, **characterized in that** the polymerization is carried out in the melt or solution by step, in the first phase of synthesis the polymerization is carried out in the melt or in solution in an organic solvent or mixture of organic solvents, preferably in a solution of benzene and / or chlorobenzene and / or toluene and / or and tetrahydrofuran, THF, at a temperature ranging from 0°C to 200°C, preferably in toluene solution at a temperature of 50°C or in melt at a temperature of 110°C, and the reaction mixture includes cyclic monomers such as lactones (preferably: ε-caprolactone, δ-valerolactone), and / or unsubstituted cyclic carbonates (preferably trimethylene carbonate) and / or derivatives of morpholine-2,5-dione (preferably morpholino-2 ,5-dione, methylmorpholine-2,5-dione) and / or dicarboxylic acid anhydrides, preferably succinic anhydride or acid anhydride maleic acid, wherein the oligomerization reaction is carried out to obtain from 50% to 100% conversion, and in this phase of the resulting oligomeric product is used in stage 2 of synthesis.

5. The method according to claim. 2, **characterized in that** the polymerization is carried out in the melt or solution by step, wherein in the first stage of the oligomer synthesis the polymerization is carried out in the presence of a small amount of an initiator or catalyst, keeping the molar ratio of the initiator to the total moles of the comonomers I/M from 1:10000 to 1:500, preferably I/M = 1:2500, the initiator or catalyst of the process is a complex compound selected from the group consisting of **monohydroxyacetylacetonate zinc (II), acetylacetonate zirconium (IV)** and / or an organic compound belonging to the group of tertiary amines or N-heterocyclic carbonates, preferably DMAP dimethylaminopyridine, 4-pyrrolidinopyridine PPY, or the process is carried out without a catalyst or initiator, and the reaction mixture further includes water and / or aqueous polyhydric alcohols selected from the group consisting of 1,4 butanediol and / or 1,3-propanediol and / or pentaerythritol and / or glycerol and / or aliphatic amines, diamines and triamines primary selected from the group consisting of hexane-1,6-diamine, octane-1,8-diamine, 1,4-diaminobutane, tetramethylene-1,4-diamine , guanidine and / or aliphatic diamine or a tri- or tetra- secondary amines selected from the group consisting of N,N'-dimethylhex-1,6-diamine, **N,N'-dimethylocto-1,8-diamine,** spermidine N-(3-aminopropyl)butane-1,4-diamine, spermine N,N'-bis(3-aminopropyl)butane-1,4-diamine, and the quantity of the alcohol and / or water and / or an amine, expressed in molar ratio of total moles of comonomers used to the sum of the moles of alcohol and / or water and / or amine in the reaction mixture is in the range from 100:1 to 2500:1.

6. The method according to claim. 2, **characterized in that** an unfavorable from the viewpoint of further use of the materials obtained material tends to flow under constant stress at work - the human body temperature, obtained amorphous copolymers or terpolymers, strongly limited by the introduction of amide groups into the chain of the oligomer synthesized in the first stage of reaction with cyclic derivatives of morpholine-2,5-dione in an amount of from 3 mol% to 25 mol% of the total content of the reaction mixture and by the input means in the first stage in the synthesis of oligomers of hydroxyl groups and /or carboxyl side groups, in the chain of synthesized oligomers by using as substrates of cyclic lactides and / or lactones and / or cyclic carbonates containing pendant hydroxyl groups and / or containing blocked chemically and / or physically hydroxyl and / or carboxyl groups, and by the synthesis of oligomers with a branched structure as a result of using in the synthesis polyhydric alcohol, especially pentaerythritol and / or glycerol and / or aliphatic amines, diamines and triamines primary especially hexane-1,6-diamine, octane-1,8-diamine, 1,4-diaminobutane, tetramethylene-1,4-diamine, guanidine and / or aliphatic diamines or a tri- or tetra- amines secondary, especially N,N'-dimethylhex-1,6-diamine, **N,N'-dimetylooktano-1,8-diamine,** spermidine N-(3-aminopropyl)butane-1,4-diamine, spermine N,N'-bis(3-aminopropyl)butane-1,4-diamine, the resulting oligomeric product is then used as the substrate in the 2-phase synthesis.

7. The method according to claim. 2, **characterized in that** the 2-phase synthesis of the melt or solution of obtained oligomers, in order to obtain the copolymers or terpolymers are added to the reaction mixture melted or dissolved cyclic monomers selected from the group consisting of lactide, especially glycolide, LL-lactide, L,D-lactide, DD-lactide) and / or lactones containing side groups in particular 5,5-dimetyloksepan-2-one, 6-(benzyloxy)oksepan-2-one, benzyl(7-oksooksepan-3-yl)acetonate) and / or cyclic carbonates containing side groups in particular 5,5-dimethyl-1,3-dioxan-2-one, 5-(benzyloxy)-1,3-dioxan-2-one, and the initiator of the copolymerization, thus starting the 2-phase synthesis, which is carried out at a temperature from 60°C to 200°C in particular in the temperature range from 80° to 120°C, using as the initiator of the reaction phase alkoxides, complexes and salts of zirconium (IV) and / or titanium (IV) particularly zirconium (IV) acetylacetonate, zirconium (IV) six-chloride, t-butoxy zirconium (IV), titanium acetylacetonate (IV).

8. The method according to claim. 2, **characterized in that** when used in phase 1 and / or 2 cyclic monomers containing few reactive side groups, after completing the 2 phases of reaction and separation and purification of the product by a chemical reaction, they are activated to the active groups hydroxyl and / or carboxyl by reduction reaction with hydrogen in the presence of a platinum catalyst particularly the reduction reaction benzyloxyls and / or benzyloacetonians groups.

9. The method according to claim. 2, **characterized in that** to enhance the intermolecular chains interactions of obtained copolymers or terpolymers and obtain long and rigid blocks in the chain structure, and thus formation of crystalline domains in the polymer material is produced, the composition of the reaction mixture in addition to the cyclic lactides and / or lactones, and / or carbonates and / or lactams include aliphatic oligoesters terminated with active group or hydroxyl groups as a chain stiffening side groups especially oligo(LL-lactide), oligo(DD-lactide), oligo(DD-lactide-co-LL-lactide, oligo(LL-lactide-co-glycolide), oligo(DD-lactide-co-glycolide) and / or the aliphatic oligocarbonates containing side groups especially oligo(5,5-dimethyl-1,3-dioxan-2-one) and / or aliphatic oligo(ester-co-carbonate), in particular oligo(3,6-dimethyl-1,4-dioxane-2,5-dione-co-5,5-dimethyl-1,3-dioxane-2-one) and / or aliphatic oligo(ester-co-amide), especially oligo(3,6-dimethyl-1,4-dioxane-2,5-dione-co-3,6-dimethylmorpholino-2,5-dione) , oligo(3,6-dimethyl-1,4-dioxane-2,5-dione-co-L-valine).

10. The method according to claim. 2 or 9, **characterized in that** to enhance the intermolecular chains interactions of obtained copolymers or terpolymers, and obtain a structure containing a long rigid blocks in the first stage of the reaction of cyclic lactide ROP with side groups particularly LL-lactide, DD-lactide and /or lactones containing side groups especially dimetylooksepan-2-one, 6-(benzyloxy)-oksepan-2-on) and / or morpholine derivatives, especially 3,6-dimetylomorpholino-2,5-dione, carried out in a solution of organic solvents, especially tetrahydrofuran, toluene, and chloroform or in a mixture of organic solvents, in particular chlorobenzene solution and / or toluene and / or tetrahydrofuran or in the melt at a temperature in the range from 0°C to 200°C especially in toluene solution at a temperature 50°C, and in the melt at 110°C oligoesters or / and oligoestercarbonates or / and oligoesteramides are obtained.

11. The method according to claim. 9 or 10, characterized that to enhance the intermolecular chains interactions of obtained copolymers or terpolymers as an initiator or catalyst for the process being conducted metallic compound is used in particular **monohydroxyacetylacetonatezinc complex (II), acetylacetonatezirconium(IV)** and / or an organic compound belonging to the group of tertiary amines or N-heterocyclic carbene especially DMAP dimethylaminopyridine, 4-pyrrolidinopyridine PPY or process without a catalyst or initiator.

12. The method according to claim. 9 or 10 or 11, that to enhance the intermolecular chains interactions of obtained copolymers or terpolymers are added to the reaction mixture in addition to water and / or aqueous polyhydric alcohols and / or mixtures thereof in particular 1,4-butanediol and / or 1,3-propanediol and / or pentaerythritol and / or glycerol and / or aliphatic primary amines and diamines, especially hexane-1,6-diamine, octane-1,8-diamine and / or secondary aliphatic diamines, preferably N,N'-dimethylhexane-1,6 -diamine, N,N'-dimethyloctane-1,8-diamine in an amount from 100:1 1 to 2000:1 molar ratio, expressed in moles of comonomers to the sum of the moles of alcohol in the reaction mixture.

13. The method according to claim. 9, **characterized in that** to enhance the intermolecular chains interactions of obtained copolymers or terpolymers and obtain a structure containing a long rigid blocks, after the conversion of the substrates in the range of 50 to 100%, in particular 98%, begins the second stage of the synthesis reaction by adding to a mixture of the products obtained in the first stage of the monomers such as cyclic lactones, especially ε-caprolactone, δ-valerolactone and / or glycolide and / or unsubstituted cyclic carbonates, in particular trimethylene carbonate and / or cyclic derivatives of morpholine-2,5-dione, particularly methylmorpholine-2,5 -dione.

14. The method according to claim. 2, **characterized in that** the second stage of the process to enhance the intermolecular chains interactions of obtained copolymers or terpolymers, after melting cyclic monomers or dissolved in solution, to the reaction mixture is added the copolymerization initiator, what begins the second phase of synthesis, which is carried out at a temperature from 60°C to 180°C in particular in the temperature range from 80°C to 120°C, as an initiator of the reaction phase alkoxides is used, complexes and salts of zinc (II), especially zinc (II) acetylacetonate monohydrate and anhydrous zinc (II) dichloride, phenylzinc (II) and / or ethylzinc (II), and / or etanolanoethylzinc (II).
